(19) Europäisches Patentamt
European Patent Office
Office européen des brevets

(11) Veröffentlichungsnummer: **0 129 211**
**B1**

# (12) EUROPÄISCHE PATENTSCHRIFT

(45) Veröffentlichungstag der Patentschrift: **04.02.87**

(51) Int. Cl.⁴: **C 07 D 265/30**, A 01 N 43/84

(21) Anmeldenummer: **84106796.0**

(22) Anmeldetag: **14.06.84**

## (54) 2,6-Trans-Dimethylmorpholinderivate und diese enthaltende Fungizide und Verfahren zur Bekämpfung von Pilzen.

(30) Priorität: **16.06.83 DE 3321712**

(43) Veröffentlichungstag der Anmeldung: **27.12.84 Patentblatt 84/52**

(45) Bekanntmachung des Hinweises auf die Patenterteilung: **04.02.87 Patentblatt 87/6**

(84) Benannte Vertragsstaaten: **AT BE CH DE FR GB IT LI NL SE**

(56) Entgegenhaltungen:
EP-A-0 019 764
DE-A-2 461 513
DE-A-2 656 747
DE-A-2 700 680
DE-A-2 752 135
DE-B-1 164 152
DE-B-1 173 722
DE-B-1 198 125
DE-B-1 214 471

(73) Patentinhaber: **BASF Aktiengesellschaft, Carl-Bosch- Strasse 38, D-6700 Ludwigshafen (DE)**

(72) Erfinder: **Buschmann, Ernst, Dr., Georg- Ludwig-Krebs- Strasse 10, D-6700 Ludwigshafen (DE)**
Erfinder: **Koenig, Karl- Heinz, Dr., Pierstrasse 8 a, D-6710 Frankenthal (DE)**
Erfinder: **Pommer, Ernst- Heinrich, Dr., Berliner Platz 7, D-6703 Limburgerhof (DE)**
Erfinder: **Drescher, Nikolaus, Dr., Brunckstrasse 31, D-6710 Frankenthal (DE)**

EP 0 129 211 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft 2,6-trans-Dimethylmorpholinderivate, diese enthaltende Fungizide und Verfahren zur Bekämpfung von Pilzen.

Es ist bekannt, N-substituierte 2,6-Dimethylmorpholine, insbesondere das Tridecyl-2,6-dimethylmorpholin (Tridemorph) als Fungizide zu verwenden. (DE 1 164 152). Die bekannten Wirkstoffe bestehen aus Gemischen der Derivate von 2,6-cis- und 2,6-trans-Dimethylmorpholin. Die Pflanzenverträglichkeit dieser Substanzen ist in vielen Fällen nicht ausreichend.

Es wurde nun gefunden, daß 2,6-trans-Dimethylmorpholinderivate der Formel

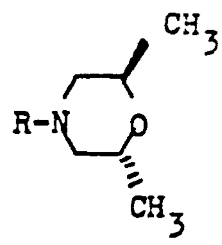

I,

in der R einen Cycloalkyl-, Cycloalkylalkyl- oder Alkylrest bedeutet, die durch ein oder mehrere $C_1$-$C_7$-Alkylreste substituiert sein können, und die Zahl der Kohlenstoffatome des Substituenten R insgesamt 7 bis 20 beträgt sowie seine N-Oxide und pflanzenverträglichen Säureadditionssalze und Metallkomplexe wesentlich besser pflanzenverträglich sind als die entsprechenden 2,6-cis-Dimethylmorpholinderivate. Die neuen 2,6-Transdimethylmorpholin-Verbindungen sind besser pflanzenverträglich als die entsprechenden bekannten cis/trans-Gemische (Tridemorph). Dies ist überraschend, weil bei dem bekannten fungiziden Handelsprodukt Fenpropimorph (DE-A 2 656 747) 4-[3-[4-(1,1-Dimethylethyl)-phenyl]-2-methyl]-propyl-2,6(cis)-dimethylmorpholin das cis-2,6-Dimethylmorpholinderivat Verwendung findet. Es wird also gegenüber dem trans-Derivat bevorzugt. Man müßte daher annehmen, daß auch bei anderen Derivaten jeweils das cis-Derivat das wertvollere der beiden Isomeren ist.

R bedeutet beispielsweise n-Nonyl, n-Decyl, n-Tridecyl, n-Dodecyl, n-$C_{14}H_{29}$, n-$C_{15}H_{31}$, n-$C_{20}H_{41}$, 1,5,9-Trimethyldecyl, 3,7,11-Trimethyldode-cyl, 3-Ethyl-1,3,7-trimethyl-octyl, 2-Methyl-tridecyl, 3,5,5-Trimethyl-hexyl, 4-Ethyl-1-methyl-octyl, 1,4-Diethyl-octyl, 2-Hexyl-hexyl, 1-Methyl-dodecyl, 2-Methyldodecyl, 2-Ethyl-5-cyclohexyl-pentyl, Cyclododecyl-methyl, 4-Ethyl-1-isobutyl-octyl, Cyclooctyl, Cyclononyl, Cyclododecyl.

N-Oxide sind z.B. N-m-Tridecyl-2,6-trans-dimethylmorpholim-N-oxid, N-Cyclo-dodecyl-2,6-trans-dimethylmorpholin-N-oxid, ein Gemisch bestehend aus N-Alkyl-2,6-trans-dimethylmorpholin-H-oxiden, wobei der Alkylrest 11 bis 14 Kohlenstoffe enthält.

Säureadditionssalze sind z.B. die Salze mit Salzsäure, HBr, $H_2SO_4$, $H_3PO_4$, Essigsäure, Propionsäure, Oxalsäure, n-Dodecylsulfonsäure, n-Dodecylphenylsulfonsäure.

Metallkomplexe sind z.B. die Komplexe mit Kupfer, Magnesium, Zink.

Die aliphatischen oder cycloaliphatischen Reste R können asymmetrische Kohlenstoffe enthalten. Die Wirkstoffe der Formel 1 können somit in Form verschiedener Emantiomere bzw. Diastereomere vorliegen. Die reinen Enantiomere und Diastereomere sowie ihre Gemische werden vom dieser Erfindung umfaßt.

Die Herstellung der trans-Verbindungen ist wie folgt möglich:

1. Auftrennung der cis-/trans-Gemische durch Dünnschichtchromatographie (n-Hexan/Aceton 9: 1 - Silicagel).

2. Destillative Trennung der cis/trans-Gemische.

3. Umsetzung von 2,6-trans-Dimethylmorpholin mit einer Verbindung der Formel RX, in der R die im Anspruch 1 genannten Bedeutungen hat und X ein Halogenatom (vorzugsweise Chlor oder Brom) bedeutet.

4. Trennung von 2,6-cis-Dimethylmorpholin und 2,6-trans-Dimethylmorpho-lin durch Destillation und anschließende Umsetzung von 2,6-trans-Di-methylmorpholin mit einer Verbindung der Formel RX oder Umsetzung mit ROH oder den entsprechenden Ketonen oder Aldehyden und Reduktion.

**Vorschrift 1**

Herstellung von Iso-Tridecylchlorid (A)

Zu 322 g $SOCl_2$ wurden 450 g Iso-Tridecanol (mit Isotridecanol bezeichnen wir ein handelsübliches Gemisch verschiedener homologer $C_{11}$-$C_{14}$-Alkohole, das 60 bis 70 % Tridecylalkohol enthält) zugetropft. Man rührte über Nacht, erwärmte 2 h auf 140°C, destillierte das Rohprodukt und erhielt 433 g farbloses Öl, Sdp. 84 bis 86°C/0,3 bar.

**Beispiel 1**

Herstellung von N-Iso-Tridecyl-2,6-trans-dimethylmorpholin (Wirkstoff Nr. 1)

30 g A und 48 g 2,6-trans-Dimethylmorpholin wurden 8 h auf 150° erwärmt. Das Rohprodukt wurde in $CH_2Cl_2$ gelöst, mit verdünnter wäßriger NaOH und anschließend mehrfach mit Wasser gewaschen. Man trocknete über $Na_2SO_4$, engte ein, destillierte und erhielt 16 g farbloses Öl, Sdp. 122° C/0,2 mbar.

**Beispiel 2**

Herstellung von N-Cyclododecyl-2,6-trans-dimethylmorpholin Wirkstoff Nr. 23)

130 g Cyclododecanon, 138 g 2,6-trans-Dimethylmorpholin und 1 g p-Toluolsulfonsäure wurden 15 h am Wasserabscheider zum Rückfluß erwärmt. Das so erhaltene Rohprodukt wurde nach Abkühlen mit Wasser gewaschen, über $MgSO_4$ getrocknet, eingeengt und destilliert. Nach nichtumgesetzten Vorprodukten destillierten 64 g (140 bis 145° C/0,3 mbar) gelbliches Öl = N-Cyclododece-nyl-2,6-trans-dimethylmorpholin (B).

Das erhaltene Enamin (B) wurde in Essigester gelöst und nach Zugabe von 5 g Pd/C-Kontakt (10 Gew.% Pd) bei 60 bis 70° C/100 bar hydriert. Danach gengte man ein und destillierte. Man erhielt 45 g farbloses Öl, Sdp. 136 bis 138° C/0,2 mbar.

In entsprechender Weise wurden die folgenden mit physikalischen Daten gekennzeichneten Verbindungen hergestellt:

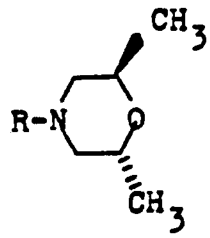

| Beispiel Nr. | R | Sdp. °C/mbar |
|---|---|---|
| 1 | Isotridecyl | 122°/0,2 |
| 2 | n-Nonyl | |
| 3 | n-Decyl | |
| 4 | n-Tridecyl | 132 - 133°/0,3 |
| 5 | n-Dodecyl | |
| 6 | n-$C_{15}H_{31}$ | |
| 7 | n-$C_{20}H_{41}$ | |
| 8 | 1,5,3-Trimethyldecyl | |
| 9 | 3,7,11-Trimethyldodecyl | |
| 10 | 3-Ethyl-1,3,7-trimethyloctyl | |
| 11 | 2-Methyltridecyl | |
| 12 | 3,5,5-Trimethylhexyl | |
| 13 | 4-Ethyl-1-methyl-octyl | |
| 14 | 1,4-Diethyl-octyl | |
| 15 | 2-Hexyl-hexyl | |
| 16 | 1-Methyldodecyl | |
| 17 | 2-Methyldodecyl | |
| 18 | 2-Ethyl-5-Cyclohexyl-pentyl | |
| 19 | Cyclododecyl-methyl | |
| 20 | 4-Ethyl-1-isobutyl-octyl | |
| 21 | Cyclooctyl | |
| 22 | Cyclononyl | |
| 23 | Cyclododecyl | 136 - 138°/0,2 |

Die neuen Verbindungen und ihre Salze, Metallkomplexverbindungen und Oxide zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffe, Bananen, Zuckerrohr, Obst und Zierpflanzen im Gartenbau, sowie Gemüse - wie Gurken, Bohnen und Kürbisgewächse-.

Die neuen Verbindungen sind insbesondere geeignet zur Bekämpfung folgender Pflanzenkrankheiten:
Erysiphe graminis (echter Mehltau) in Getreide,
Erysiphe cichoriacearum (echter Methltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Mycosphaerella musicola an Bananen,
Corticium salmonicolor an Hevea,
Ganoderma pseudoferreum an Hevea und
Exobasidium vexans an Tee.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfomate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch im Materialschutz u.a. zur Bekämpfung holzzerstörender Pilze, wie Coniophora puteana und Polystictus versicolor eingesetzt werden. Die Wirkstoffe eignen sich auch zur Behandlung von Früchten gegen Schimmelpilzbefall. Ferner können sie in Verbindung mit Kunststoffdispersionen zu Wundbehandlungen gegen pilzliche Infektionen verwendet werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl-alphapyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 4 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung des Beispiels 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 20 Gewichtsteile der Verbindung des Beispiels 2 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitlauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung 4 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 1 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine verdünnte wäßrige Dispersion.

IX. 20 Teile der Verbindung des Beispiels 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die neuen Mittel kömnen in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den neuen Verbindungen kombiniert werden können, sind beispielsweise:

Schwefel,

Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbanat oder Zinkethylenbisdithiocarbamat, Tetramethylthiuramdisulfide,

Ammoniak-Komplex von Zink-(N,N-ethylen-bis-dithiocarbamat) und N,N'-Polyethylen-bis-(thiocarbamoyl)-disulfid, Zink-(N,N'-propylen-bis-dithiocarbanat), Ammomiak-Komplex von Zink-(N,N'-propylen-bis-dithiocarbanat) und N,N'-Propylen-bis(thiocarbamoyl)-disulfid;

Nitroderivate, wie

Dinitro-(l-methylheptyl)-phenylcrotonat,

2-sec.-Butyl-4,6-dinitrophenyl-3,3-dimethylacrylat,

2-sec.-Butyl-4,6-dinitrophenyl-isopropylcarbonat;

heterocyclische Substanzen, wie

N-Trichlormethylthio-tetrahydrophthalimid,

N-(1,1,2,2-Tetrachlorethylthio)-tetrahydrophthalimid,

N-Trichlormethylthio-phthalimid,

2-Heptadecyl-2-imidazolin-acetat,

2,4-Dichlor-6-(o-chloranilino)-s-triazin,

0,0-Diethyl-phthalimidophosphonothioat,

5-Amino-1-(bis-(dimethylamino)-phosphinyl)-3-phenyl-1,2,4-triazol,

5-Ethoxy-3-trichlormethyl-1,2,4-thiadiazol,

2,3-Dicyano-1,4-dithioanthrachinon,

2-Thio-1,3-dithio-(4,5-b)-chinoxalin,

1-(Butylcarbamoyl)-2-benzimidazol-carbaminsäuremethylester,

2-Methoxycarbonylamino-benzimidazol,

2-Thodanmethylthio-benzthiazol,

4-(2-Chlorphenylhydrazono)-3-methyl-5-isoxazolon,

Pyridin-2-thiol-1-oxid,

8-Hydroxychinolin bzw. dessen Kupfersalz,

2,3-Dihydro-5-carboxanilido-6-methyl-1,4-oxathiin-4,4-dioxid,

2,3-Dihydro-5-carboxanilido-6-methyl-1,4-oxathiin,

2-(Furyl)-benzimidazol,

Piperazin-1,4-diyl-bis-(1-(2,2,2-trichlor-ethyl)-formamid),

2-(Thiazolyl-(4))-benzimidazol,

5-Butyl-2-dimethylamino-4-hydroxy-6-methyl-pyrimidin,

Bis-(p-Chlorphenyl)-pyridinmethanol,

1,2-Bis-(3-ethoxycarbonyl-2-thioureido)-benzol,

1,2-Bis-(3-methoxycarbonyl-2-thioureido)-benzol

und weitere Substanzen, wie

Dodecylguamidinacetat,

3-(3-(3,5-Dimethyl-2-oxycyclohexyl)-2-hydroxyethyl)-glutaramid,

Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloramilino)-l-formylamino-2,2,2-trichlorethan,
DL-Methyl-N-2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-l')-4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Der folgende Versuch zeigt die biologische Wirksamkeit gegen echten Mehltau (Erysiphe graminis var. hordei) und insbesondere die Pflanzenverträglichkeit der neuen N-substituierten 2,6-trans-Dimethylmorpholine.

**Versuch**

Gerstenkeimlinge (Hauters Pfälzer Sommergerste) wurden im Einblattstadium mit wäßriger Wirkstofflösung verschiedener Konzentration besprüht. 24 Stunden nach der Behandlung erfolgte die künstliche Beimpfung mit Sporen des Gerstenmehltaus (Erysiphe graminis var. hordei). Die Versuchsauswertung wurde 10 Tage nach der Infektion vorgenommen.

Das Ergebnis des Versuches zeigt, daß der Wirkstoff 1 bei der Anwendung als 0,C025; 0,005; 0,01 und 0,02 %ige (Gew.%) Spritzbrühe geringere Blattschäden (I) erzeugt als die Verbindung N-Isotridecyl-2,6-cis-dimethylmorpholin (2) und die Verbindung Tridemorph.

**Patentansprüche** für die Vertragsstaaten BE CH DE FR GB IT LI NL SE

1. 2,6-trans-Dimethylmorpholinderivat der Formel

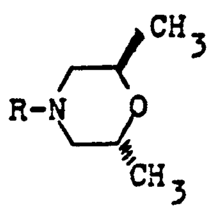 I,

in der R einen Cycloalkyl-, Cycloalkylalkyl- oder Alkylrest bedeutet, die durch ein oder mehrere $C_1$-$C_7$-Alkylreste substituiert sein können, und die Zahl der Kohlenstoffatome des Substituenten R insgesamt 7 bis 20 beträgt sowie seine N-Oxide und pflanzenverträglichen Säureadditionssalze und Metallkomplexe.

2. Fungizid enthaltend eine Verbindung der Formel I gemäß Anspruch 1.

3. Fungizid enthaltend eine Verbindung der Formel I gemäß Anspruch 1 und einen festen oder flüssigen Trägerstoff.

4. Verfahren zur Bekämpfung von Pilzen dadurch gekennzeichnet, daß man eine fungizid wirksame Menge einer Verbindung der Formel 1 gemäß Anspruch 1 auf durch Pilzbefall bedrohte Materialien, Flächen, Pflanzen oder Saatgüter oder auf die Pilze einwirken läßt.

5. Verfahren zur Herstellung von 2,6-trans-Dimethylmorpholinen, wie im Anspruch 1 definiert, dadurch gekennzeichnet, daß man eine Verbindung der Formel RX, in der R die obengenannten Bedeutungen hat und X ein Halogenatom bedeutet, mit 2,6-trans-Dimethylmorpholin umsetzt.

6. 2,6-trans-Dimethylmorpholinderivat gemäß Anspruch 1 nämlich N-n-Tridecyl-2,6-trans-dimethylmorpholin, N-n-Dodecyl-2,6-trans-di-methyl-morpholin, N-Cyclododecyl-2,6-trans-dimethyl-morpholin oder ein Gemisch bestehend aus N-Alkyl-2,6-trans-dimethyl-morpholinen, wobei der Alkylrest gegebenenfalls verzweigt ist und jeweils 11 bis 14 Kohlenstoffatome enthält.

7. Fungizides Mittel enthaltend ein 2,6-trans-Dimethylmorpholinderivat gemäß Anspruch 1, nämlich N-n-Tridecyl-2,6-trans-dimethyl-morpholin, N-n-Dodecyl-2,6-trans-dimethyl-morpholin, N-Cyclododecyl-2,6-trans-dimethyl-morpholin oder ein Gemisch bestehend aus N-Alkyl-2,6-trans-dimethyl-morpholinen, wobei der Alkylrest gegebenenfalls verzweigt ist und jeweils 11 bis 14 Kohlenstoffatome enthält.

**Patentansprüche** für den Vertragsstaat AT

1. Fungizid enthaltend ein 2,6-trans-Dimethylmorpholinderivat der Formel

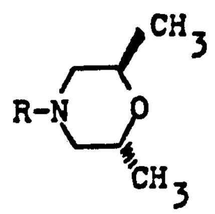

I,

in der R einen Cycloalkyl-, Cycloalkylalkyl- ode; Alkylrest bedeutet, die durch ein oder mehrere $C_1$-$C_7$ Alkylreste substituiert sein können, und die Zahl der Kohlenstoffatome des Substituenten R insgesamt 7 bis 20 beträgt sowie seine N-Oxide und pflanzenverträglichen Säureadditionssalze und Metallkomplexe.

2. Fungizid enthsltend eine Verbindung der Formel 1 gemäß Anspruch 1 und einen festen oder flüssigen Trägerstoff.

3. Verfahren zur Bekämpfung von Pilzen dadurch gekennzeichnet, daß man eine fungizid wirksame Menge einer Verbindung der Formel 1 gemäß Anspruch 1 auf durch Pilzbefall bedrohte Materialien, Flächen, Pflanzen oder Saatgüter oder auf die Pilze einwirken läßt.

4. Verfahren zur Herstellung von 2,6-trans-Dimethylmorpholinen, wie im Anspruch 1 definiert, dadurch gekennzeichnet, daß man eine Verbindung der Formel RX, in der R die obengenannten Bedeutungen hat und X ein Halogenatom bedeutet, mit 2,6-trans-Dimethylmorpholin umsetzt.

5. Fungizides Mittel enthaltend ein 2,6-trans-Dimethylmorpholinderivat gemäß Anspruch 1, nämlich N-n-Tridecyl-2,6-trans-dimethyl-morpholin, N-n-Dodecyl-2,6-trans-dimethyl-morpholin, N-Cyclododecyl-2,6-trans-dimethyl-morpholin oder ein Gemisch bestehend aus N-Alkyl-2,6-trans-dimethyl-morpholinen, wobei der Alkylrest gegebenenfalls verzweigt ist und jeweils 11 bis 14 Kohlenstoffatome enthält.

**Claims** for the contracting states: BE, CH, DE, FR, GB, IT, LI, NL, SE

1. A 2,6-trans-dimethylmorpholine derivative of the formula

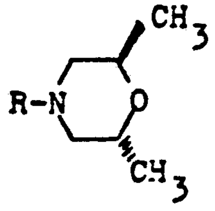

I,

where R is a Cycloalkyl, Cycloalkylalkyl or alkyl radical which can be substituted by one or more $C_1$-$C_7$-alkyl radicals, and the total number of carbon atoms in the substituent R is from 7 to 20, and its N-oxides and plant-tolerated addition salts with acids and metal complexes.

2. A fungicide containing a compound of the formula I as claimed in claim 1.

3. A fungicide containing a compound of the formula I as claimed in claim 1 and a solid or liquid carrier.

4. A process for combatting fungi, wherein a fungicidally effective amount of a compound of the formula I as

claimed in claim 1 is allowed to act on the materials, areas, plants and seed threatened by fungal attack, or on the fungi themselves.

5. A process for the preparation of a 2,6-trans-morpholine derivative as defined in Claim 1, wherein a compound of the formula RX, where R has the above meanings and X is halogen, is reacted with 2,6-trans-dimethylmorpholine.

6. A 2,6-trans-morpholine derivative as Claimed in Claim 1, namely N-n-tridecyl-2,6-trans-dimethylmorpholine, N-n-dodecyl-2,6-trans-di-methylmorpholine, H-Cyclodecyl-2,6-trans-dimethylmorpholine or a mixture of N-alkyl-2,6-trans-dimethylmorpholines, where the alkyls may or may not be branched and each contain 11 to 10 carbon atoms.

7. A fungicidal agent containing a 2,6-trans-morpholine derivative as claimed in claim 1, namely N-n-tridecyl-2,6-trans-dimethylmorpholine, N-n-dodecyl-2,6-trans-dimethylmorpholine, N-cyclodecyl-2,6-trans-dimethylmorpholine or a mixture of N-alkyl-2,6-trans-dimethylmorpholines, where the alkyls may or may not be branched and each contain 11 to 10 carbon atoms.

**Claims** for the contracting state: AT

1. A fungicide containing a 2,6-trans-dimethylmorpholine derivative of the formula

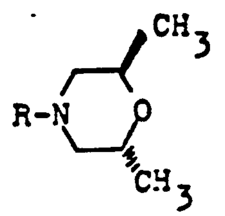

I,

where R is a cycloalkyl, cycloalkylalkyl or alkyl radical which can be substituted by one or more $C_1$-$C_7$-alkyl radicals, and the total number of carbon atoms in the substituent R is from 7 to 20, and its N-oxides and plant-tolerated addition salts with acids and metal complexes.

2. A fungicide containing a compound of the formula I as claimed in claim 1 and a solid or liquid carrier.

3. A process for combatting fungi, wherein a fungicidally effective amount of a compound of the formula I as defined in claim 1 is allowed to act on the materials, areas, plants and seed threatened by fungal attack, or on the fungi themselves.

4. A process for the preparation of a 2,6-trans-morpholine derivative as defined in claim 1, wherein a compound of the formula RX, where R has the above meanings and X is halogen, is reacted with 2,6-trans-dimethylmorpholine.

5. A fungicidal agent containing a 2,6-trans-morpholine derivative as defined in claim 1, namely N-n-tridecyl-2,6-trans-dimethylmorpholine, N-n-dodecyl-2,6-trans-dimethylmorpholine, N-cyclodecyl-2,6-trans-dimethylmorpholine or a mixture of N-alkyl-2,6-trans-dimethylmorpholines, where the alkyls may or may not be branched and each contain 11 to 14 carbon atoms.

**Revendications** pour les Etats contractants: BE, CH, DE, FR, GB, IT, LI, NL, SE

1. Dérivé de 2,6-trans-diméthylmorpholine de formule

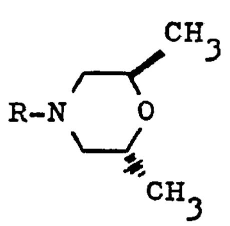

I,

dans laquelle R représente un reste cycloalkyle, cycloalkylalkyle ou alkyle, qui peut être substitué par un ou plusieurs restes alkyle en $C_1$-$C_7$, et le nombre des atomes de carbone du substituant R est au total de 7 à 20, ainsi que ses N-oxydes et ses sels d'addition d'acides et complexes métalliques acceptables pour les plantes.

2. Fongicide contenant un composé de formule I selon la revendication 1.

3. Fongicide contenant un composé de formule I selon la revendication 1 et un véhicule solide ou liquide.

4. Procédé de lutte contre les champignons, caractérisé par le fait que l'on fait agir une quantité, efficace du point de vue fongicide, d'un composé de formule I selon la revendication 1 sur les matériaux, surfaces, plantes, ou semences menacés par une attaque de champignone ou sur les champignons eux-mêmes.

5. Procédé de préparation de 2,6-trans-diméthylmorpholines telles que définies dans la revendication 1, caractérisé par le fait que l'on fait réagir un composé de formule RX, dans laquelle R a les significations sus-indiquées, et X représente un atome d'halogène, avec de la 2,6-trans-diméthylmorpholine.

6.- Dérivé de 2,6-trans-diméthylmorpholine selon la revendication 1, notamment N-n-tridécyl-2,6-trane-diméthylmorpholine, N-n-dodécyl-2,6-trans-diméthyl-morpholine, N-Cyclododécyl-2,6-trans-diméthyl-morpholine ou un mélange constitué de N-alkyl-2,6-trans-diméthyl-morpholines, le reste alkyle étant éventuellement ramifié et contenant chacun 11 à 14 atomes de carbone.

7.- Agent fongicide contenant un dérivé de 2,6-trans-diméthylmorpholine selon la revendication 1, notamment N-n-tridécyl-2,6-trans-diméthyl-morpholine, N-n-dodécyl-2,6-trans-diméthyl-morpholine, N-cyclododécyl-2,6-trans-diméthyl-morpholine, ou un mélange constitué de N-alkyl-2,6-trans-diméthyl-morpholines, le reste alkyle étant éventuellement ramifié et contenent chacun 11 à 14 atomes de carbone.

**Revendications** pour l'Etat contractant: AT

1. Fongicide contenant un dérivé de 2,6-trans-diméthyl-morpholine de formule

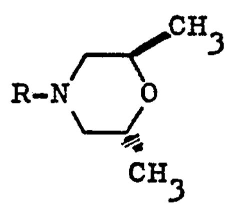

I,

dans laquelle R représente un reste cycloalkyle, cycloalkylalkyle ou alkyle, qui peut être substitué par un ou plusieurs restes alkyle en $C_1$-$C_7$, et le nombre des atomes de carbone du substituant R est au total de 7 à 20, ainsi que ses N-oxydes et ses sels d'addition d'acides et complexes métalliques acceptables pour les plantes.

2. Fongicide contenant un composé de formule 1 selon la revendication 1 et un véhicule solide ou liquide.

3. Procédé de lutte contre les champignons, caractérisé par le fait que l'on fait agir une quantité, efficace du point de vue fongicide, d'un composé de formule 1 selon la revendication 1 sur les matériaux, surfaces, plantes, ou semences menacés par une attaque de champignons ou sur les champignons eux-mêmes.

4. Procédé de préparation de 2,6-trans-diméthylmorpholines telles que définies dans la revendication 1, caractérisé par le fait que l'on fait réagir un composé de formule RX, dans laquelle R a les significations sus-indiquées, et X représente un atome d'halogène, avec de la 2,6-trans-diméthylmorpholine.

5.- Agent fongicide contenent un dérivé de 2,6-trana-diméthylmorpholine selon la revendication 1, notamment N-n-tridécyl-2,6-trans-diméthyl-morpholine, N-n-dodécyl-2,6-trane-diméthyl-morpholine, N-cyclododécyl-2,6-trans-diméthyl-morpholine, ou un mélange constitué de N-alkyl-2,6-trans-diméthyl-morpholines, le reste alkyle étant éventuellement ramifié et contenant chacun 11 à 14 atomes de carbone.